# EUROPEAN PATENT SPECIFICATION

(11) **EP 3 235 929 B1**
(45) Date of publication and mention of the grant of the patent: **19.08.2020**
(21) Application number: 15870242.3
(22) Date of filing: 10.12.2015
(51) Int. Cl.: D01F 1/10, D01F 2/00

(54) **SYNTHETIC RESIN AND SYNTHETIC FIBER CONTAINING LINOLENIC ACID, AND METHOD FOR PRODUCING SAME**
KUNSTHARZ UND KUNSTFASERN MIT LINOLENSÄURE UND VERFAHREN ZUR HERSTELLUNG DAVON
RÉSINE SYNTHÉTIQUE ET FIBRE SYNTHÉTIQUE CONTENANT DE L'ACIDE LINOLÉNIQUE, ET LEUR PROCÉDÉ DE FABRICATION

(30) Priority: 16.12.2014 KR 20140181298
(43) Date of publication of application: 25.10.2017
(73) Proprietor: Park, Hee Seoup, Seoul 03009 (KR); Kim, Ki Suk, Daegu 41586 (KR)
(72) Inventor: Park, Hee Seoup, Seoul 03009 (KR); Kim, Ki Suk, Daegu 41586 (KR)
(74) Representative: Zacco Sweden AB
(86) International application number: PCT/KR2015/013505
(87) International publication number: WO 2016/099075

(56) References cited:
- EP-A1- 1 443 070
- KR-A- 940 014 558
- KR-B1- 100 830 093
- US-A1- 2009 226 593
- US-A1- 2012 328 804
- US-A1- 2013 053 479
- US-A1- 2013 053 480
- US-A1- 2013 203 919

## Description

### Technical Field

The present invention relates to a synthetic resin and synthetic fiber containing linolenic acid, and to a method of manufacturing the same. More particularly, the present invention relates to a synthetic resin and synthetic fiber containing linolenic acid, which are superior in various properties such as strength, elongation, etc. and may exhibit a good outer appearance and significantly increased antimicrobial activity, and to a method of manufacturing the same.

### Background Art

A synthetic resin, especially a polyester resin, is superior in various properties such as strength and elongation and is inexpensive, and is thus widely useful as clothing and other industrial materials. However, it is excessively hard and causes discomfort when touching the skin, and may severely generate static electricity upon the production or use thereof.

Many attempts have been made to solve the above problems, and may include, for example, fixing methods by directly applying a plant extract onto a synthetic fiber, as disclosed in Korean Patent Nos. 10-0726409 and 10-0515808. However, the synthetic fiber thus manufactured is problematic in that the applied plant extract may be stripped during the washing process, making it difficult to exhibit persistent effects.

With the goal of solving these problems, there have been devised methods of attaching a plant extract in the form of microcapsules to the surface of a fiber. However, such methods are problematic in that when the fiber thus manufactured is subjected to friction, washing, or photoexposure, the microcapsules attached thereto may be easily detached.

In order to manufacture a fiber containing a plant extract, a melt-spinning process using a plant extract may be taken into consideration, but the melt spinning is performed at a high temperature of 200°C or more, and thus production itself is impossible due to volatilization, decomposition and deterioration of the plant extract that is used, or the properties of the obtained fiber may become very poor.

In this regard, Korean Patent No. 10-0910241 is provided.

This technique discloses a nanofiber containing a natural vegetable extract or natural vegetable essential oil manufactured by electrospinning a spinning solution comprising (a) at least one of a natural vegetable extract and natural vegetable essential oil, (b) at least one fiber-forming polymer, and (c) a solvent for dissolving the components (a) and (b).

The electrospinning process is performed in a manner in which a solution discharged via a nozzle is converted into a jet stream by means of an electric force formed between a collector and the nozzle, after which the solvent contained in the jet stream is volatilized after reaching the incomplete zone, thereby forming a pure nanofiber.

By virtue of the above process, most of the problems occurring in conventional techniques have been solved. However, the fiber thus obtained is unable to achieve an enhancement in strength through the molecular orientation of the polymer alone, whereby various properties thereof may deteriorate, making it impossible to use it as a clothing material requiring predetermined basic properties.

Meanwhile, Korean Patent No. 10-0563560 discloses a vegetable protein synthetic fiber comprising a vegetable protein and polyvinyl alcohol, wherein the vegetable protein is used in an amount of 5 to 23 parts by weight and the polyvinyl alcohol is used in an amount of 77 to 95 parts by weight based on the total weight of these two components. The protein that is used is obtained by subjecting beans, peanuts, cotton seeds, etc. to wet milling to separate and extract a protein, which is then crushed, degreased and immersed.

The fiber thus obtained may exhibit superior air permeability and characteristics similar to those of cashmere, which is a high-grade wool material. However, this fiber is very poor in strength and durability and is thus unsuitable for use in clothing.

With the goal of solving the above problems, Korean Patent No. 10-1171947 is provided.

This technique discloses a method of manufacturing a synthetic fiber containing a vegetable fatty acid by adding a fiber-forming polymer material selected from among polyester-, nylon- and polypropylene-based resins with 0.1 to 10.0 wt% of a vegetable fatty acid and then performing a typical melt-spinning process.

The fiber thus obtained may exhibit superior basic properties such as strength and elongation, a good outer appearance, and excellent antimicrobial activity, color depth and antistatic properties, and is thus suitable for use in a high-grade clothing material.

However, this technique has many problems as follows.
(1) During the production process, yarn breakage may occur or back-pressure hunting of a spinneret may be frequently caused, making it difficult to realize mass production, undesirably resulting in excessively high manufacturing costs.
(2) The quality of the obtained fiber is not uniform, and particularly the functionality of the obtained product, such as antimicrobial activity or the like, is not uniform.
(3) The quality of the obtained fiber varies greatly depending on the kind of fatty acid that is used.

### Disclosure

### Technical Problem

Accordingly, the present invention has been made keeping in mind the above problems encountered in the related art, and the present invention is intended to provide a synthetic resin and synthetic fiber containing vegetable oil, which facilitate mass production and the manufacturing costs of which are low. In addition, the present invention is intended to provide a synthetic resin and synthetic fiber containing vegetable oil, in which the quality and functionality of the obtained fiber are uniform. In addition, the present invention is intended to provide a synthetic resin and synthetic fiber, the functionality of which may be exhibited regardless of the kind of vegetable oil that is used.

### Technical Solution

The present invention provides a synthetic resin or synthetic fiber containing 0.7 to 2.5 wt% of a vegetable oil that comprises 70 wt% or more of linolenic acid and a low-molecular-weight volatile material in an amount of less than 5.0 wt%. In addition, the present invention provides a method of manufacturing a synthetic fiber, comprising: adding 0.7 to 2.5 wt% of a vegetable oil that comprises 70 wt% or more of linolenic acid and a low-molecular-weight volatile material in an amount of less than 5.0 wt% to a fiber-forming polymer material and then performing melt spinning. In addition, the present invention provides a method of manufacturing a synthetic resin, comprising: polymerizing a fiber-forming polymer material, adding 0.7 to 2.5 wt% of a vegetable oil that comprises 70 wt% or more of linolenic acid and a low-molecular-weight volatile material in an amount of less than 5.0 wt%, and performing graft polymerization.

Hereinafter, a detailed description will be given of the present invention.

Examples of commonly useful vegetable oil may include corn oil, soybean oil, rapeseed oil, grape seed oil, linseed oil, sesame oil, olive oil, perilla oil, walnut oil, cold peanut oil, sunflower oil, safflower oil, cottonseed oil, palm oil, red pepper seed oil, rice bran oil, pumpkin seed oil, green tea seed oil, almond oil, pine nut oil, and evening primrose oil.

The fatty acid contained therein may include an unsaturated fatty acid, such as oleic acid, linoleic acid, linolenic acid, stearic acid, palmitic acid, licanic acid, and ricinoleic acid, and about 5 to 30% of any type of saturated fatty acid.

The composition of chia seed oil is shown in Table 1 below.

**[Table 1]**

| Classification | per 100 g |
|---|---|
| Total fat (g) | 95.33 |
| Saturated fat (g) | 10.78 |
| Trans fatty acid (g) | 0.00 |
| Monounsaturated fat (g) | 7.35 |
| Polyunsaturated fat (g) | 77.2 |
| Linolenic acid (g) | 59.32 |
| Linoleic acid (g) | 17.88 |

The composition of linseed oil is shown in Table 2 below.

**[Table 2]**

| Component | Amount (g/100 g fatty acid) | Note |
|---|---|---|
| Myristic acid | 0.04021 | |
| Pentadecanoic acid | 0.02228 | |
| Palmitic acid | 5.27593 | |
| Palmitoleic acid | 0.05897 | |
| Margaric acid | 0.06364 | |
| Heptadecenoic acid | 0.04187 | |
| Stearic acid | 3.47834 | |
| Oleic acid | 18.56481 | |
| Linoleic acid | 15.39735 | |
| Linolenic acid | 56.41282 | |
| Arachidic acid | 0.14637 | |
| Gadoleic acid | 0.13117 | |
| Eicosadienoic acid | 0.04389 | |
| Eicosadienoic acid | 0.02286 | |
| Heneicosadienoic acid | 0.04995 | |
| Behenic acid | 0.12625 | |
| Erucic acid | 0.01942 | |
| Lionaceric acid | 0.10388 | |

Among many kinds of vegetable oil and many fatty acids thereof, whether any component imparts good functionality to the produced fiber or does not cause trouble during the production of the fiber is of interest. Through various studies, the present inventors have ascertained that linolenic acid, among vegetable fatty acids, significantly contributes to the functionality and productivity of a fiber, and also that the quality of the obtained fiber may become uniform when the amount of linolenic acid is a predetermined level or more.

Furthermore, when the amount of the low-molecular-weight volatile material is less than 5 wt%, preferably less than 3 wt%, more preferably less than 1 wt%, and still more preferably less than 0.1 wt%, mass production of the fiber can be found to be possible. If the amount of the low-molecular-weight volatile material exceeds the above range, problems such as yarn breakage may occur upon fiber production. As used herein, the term "low-molecular-weight volatile material" refers to a material having a boiling point of 120 to 220°C. Such a material may cause yarn breakage without participating in chemical bonding with the fiber-forming polymer material.

In the usable vegetable oil, the amount of linolenic acid is 70 wt% or more, preferably 75 wt% or more, more preferably 80 wt% or more, and still more preferably 82 wt% or more. If the amount of linolenic acid in the vegetable oil is less than the above range, troubles may occur upon production of the fiber, and furthermore, the functionality of the obtained fiber is unsatisfactory.

Vegetable oil that is useful in the present invention contains 50% or more of linolenic acid. If the amount of linolenic acid is less than the above range, the amount thereof cannot be increased to 70% or more even through any process such as purification, as will be described later.

Examples of vegetable oil containing 50% or more of linolenic acid may include chia seed oil, linseed oil, perilla oil, etc. The amount of linolenic acid in chia seed oil is about 58 to 62%, and the amount of linolenic acid in linseed oil or perilla oil is about 50 to 57%.

As for chia seed oil, *Salvia hispanica* or *Salvia columbariae* is a kind of salvia belonging to the genus *Perilla frutescens* of the Mint family and an annual subtropical plant (Hickman, James C. The Jepson Manual Higher Plants of California. University of California Press, Berkeley Los Angeles London. (1993), USDA Natural resources Conservation Service, [web application]. 2006), and originates from Mexico and is cultivated for commercial purposes in the central plateau of Argentina, Bolivia, Ecuador and Guatemala.

Chia seed has been used for promotion of vitality, fitness, health, and beauty in Latin America including Mexico for 1000 years, and has traditionally been utilized in areas such as Mexico but is not widely known in Asia or Europe. Such chia seed contains about 32% vegetable fat, linolenic acid constituting about 60% thereof.

In order to use the above oil in the present invention, the amount of linolenic acid has to be increased to 70% or more.

Here, the amount of linolenic acid in the vegetable oil may be increased through various methods. Specifically, a saturated fatty acid of the vegetable oil is removed or the amount thereof is reduced. A saturated fatty acid constitutes about 5 to 30% of the vegetable oil.

An example of the method of removing the saturated fatty acid from the vegetable oil is disclosed in Korean Patent No. 10-0741406.

This method is described below:
a) subjecting a vegetable edible oil to transesterification with C1-8 alkanol in the presence of an alkali metal or alkaline earth metal C1-8 alkoxide catalyst, performing neutralization with an organic acid or inorganic acid, and removing the alkanol, thereby obtaining a saturated/unsaturated fatty acid-alkyl ester mixture;
b) reacting the fatty acid-alkyl ester mixture obtained in a) with urea dissolved in C1-8 alkanol, whereby the saturated fatty acid alkyl ester-urea molecular composite is precipitated in crystalline form and removed;
c) mixing 3 mol of the unsaturated fatty acid alkyl ester separated in b) with a triglyceride comprising C1-8 fatty acid having a low molecular weight and then performing interesterification in the presence of an alkali metal or alkaline earth metal alkoxide catalyst, thereby obtaining a vegetable edible oil composed exclusively of unsaturated fatty acid, the triglyceride structure of which is reconstructed.

When this method is adopted, the saturated fatty acid is completely removed. In the case of linseed oil or perilla oil, the amount of linolenic acid is increased to about 70%, and in the case of chia seed oil, the amount of linolenic acid exceeds 70%.

Even when the amount of linolenic acid is increased in this way, it is necessary to remove the low-molecular-weight volatile material or to reduce the amount thereof. Here, the low-molecular-weight volatile material indicates a volatile material having a boiling point of about 120 to 220°C. Such a volatile material insignificantly contributes to an improvement in the functionality of the obtained fiber and causes trouble during the production process, and thus it is preferable to remove as much thereof as possible.

As for the removal process, the vegetable oil is heated to 120 to 250°C for a time period ranging from 20 min to 3 hr. The heating process is of a non-contact type, and may be discontinuous or continuous. Here, it is noted that functional linolenic acid should be prevented from deterioration or acidification due to heat upon the heating process. Even by this process, the low-molecular-weight volatile material is removed, whereby the amount of linolenic acid is increased somewhat.

An alternative to the removal of the low-molecular-weight volatile material or high-viscosity material includes a supercritical removal process using carbon dioxide. Since carbon dioxide is used as the inert solvent, the risk of deterioration or acidification is prevented and the volatile material may be removed at lower temperatures under high-pressure conditions.

As mentioned above, the vegetable oil, in which the amount of linolenic acid is increased and the amount of low-molecular-weight volatile material is reduced, may be effectively added during the production process, and the appropriate amount thereof that is added is 0.7 to 2.5 wt%. If the amount thereof is less than the above lower limit, the addition effect may become poor. On the other hand, if the amount thereof exceeds the above upper limit, the effect is not further improved but the production process begins to become troublesome, undesirably increasing the production costs.

### Advantageous Effects

According to the present invention, the fiber can provide a fabric, which exhibits superior dyeing and color depth properties, high dye fastness, and good elasticity and tactile sensation. Also, it has excellent softness and can thus manifest a sufficient weight-reducing effect in a lightweight product requiring softness even under weight-reducing conditions corresponding to 1/4 or less of a typical polyester fiber, thus remarkably decreasing production costs.

### Mode for Invention

Hereinafter, a detailed description will be given of the present invention.

In the following Examples and Comparative Examples, various properties are measured as follows.

### * Measurement of fat content (wt%)

Through GC/MS chromatogram and Gas chromatograph/Mass spectrometer of vegetable oil, measurement was performed based on GC-FID of main products.

### * Intrinsic viscosity of polyester (dl/g)

Using a mixed solvent of phenol/tetrachloroethane, the conversion viscosity at 30°C was measured and calculated as a function of concentration, and the conversion viscosity at which the concentration is zero was represented by IV.

### * Melting point, glass transition temperature, crystallinity (J/g), crystallization rate (%)

Using a differential scanning calorimeter [DSC (200F3, NETZSCH)], a sample was tested at heating and cooling rates of 10 °C/min, and thus the crystallinity (J/g) was determined and the crystallization rate relative to a perfect crystal was determined.

### * Strength (g/d), elongation (%), dyeing rate

Using a tension tester by KSF 0520 and through a dyeing process according to KSK ISO/05-A06, comparison with a standard color was performed.

### * Wet-heat shrinkage rate of long polyester fiber

A long-fiber sample was separated to a length of 50 cm under a load of 1 mg/d, immersed in pure water at 98°C for 30 min and then taken out of the water, after which the shrunken length was measured and the shrinkage rate relative to the initial length of 50 cm was calculated.

### * Perspiration-absorbing and quick-drying rate of fiber

According to KSK 0815, the absorption rate, absorption speed score and drying speed were compared.

### (Comparative Example 1)

A mixture of pure polyterephthalic acid and ethylene glycol at a weight ratio of 0.86:0.33 (totaling 1.19) was prepared, esterified at a high temperature of 220°C or more and a pressure of 1.5 kg/m² or more to evaporate water at a weight ratio of 0.19, and polymerized at a high temperature of 280°C or more in a vacuum, thus giving a polyester polymer (PETI) having a polymerization degree of about 25, which was then cooled and solidified, thereby producing a pellet-shaped raw chip (PET RC). The PET RC was primarily dried at 105°C and secondarily dried using hot air at 145 to 150°C, thus obtaining a PET dry chip (PET DC) with IV of 0.640 to 0.650.

To the PET DC, a vegetable fatty acid containing 67 wt% of linolenic acid and about 3.2 wt% of a low-molecular-weight volatile material, obtained from commercially available linseed oil using the method disclosed in Korean Patent No. 0741406, was uniformly continuously sprayed in an amount of 0.6 to 1.2% using a quantitative pump, followed by melt-extrusion using a 5-stage melt extruder, spinning using a spinneret having a diameter of 0.17 to 0.23, and then quick cooling, thereby producing a long polyester fiber.

Upon stretching and winding at 3100 to 3400 m/min using a lower winder, POY was produced, and SDY was obtained upon stretching and winding at 3800 to 5500 m/min.

The POY and SDY in which the vegetable fatty acid was crosslinked and polymerized exhibited strength similar to that of general yarn, and were increased by about 4% in elongation. However, when 0.8 wt% or more of fatty acid was added, back-pressure hunting of the spinneret and single-yarn breakage at the spinneret outlet occurred significantly.

The properties of the obtained yarn are described below.

The results of DSC and crystallization rate are shown in Table 3 below.

**[Table 3]**

| | DSC Results | | | Crystallization rate | |
|---|---|---|---|---|---|
| Classification | Tg (°C) | Tc (°C) | Tm (°C) | POY (%) | DTY (%) |
| General yarn | 76 | 152 | 254 | 34.6 | 30.6 |
| Fatty acid-containing yarn (0.8%) | 82 | 154 | 253 | 29.1 | 26.6 |

The physical properties (POY 124De) are shown in Table 4 below.

**[Table 4]**

| | Strength (g/d) | Elongation (%) | Tension upon 1.5-fold stretching (TN 1.5-fold) | Wet-heat shrinkage rate after 1.7-fold stretching (BWS) |
|---|---|---|---|---|
| General yarn | 2.76 | 120 | 287 | 7.2 |
| Fatty acid-containing yarn (0.8%) | 2.76 | 126 | 246 | 8.0 |

The physical properties (DTY 75De) are shown in Table 5 below.

**[Table 5]**

| | Strength (g/d) | Elongation (%) | Bulk | Dyeing (%) | Perspiration-absorbing and quick-drying | |
|---|---|---|---|---|---|---|
| | | | | | Drying speed (min) | Absorption speed (mm/10 min) |
| General yarn | 4.92 | 18 | 38% | 8 | 240 | 100 |
| Fatty acid-containing yarn (0.8%) | 4.82 | 21 | 38.5% | 13 | 210 | 130 |

### (Example 1)

A preconcentrated vegetable fatty acid having about 67 wt% of linolenic acid was added with 1.0 wt% of an antioxidant, subjected to primary distillation including distillation for 20 min each in individual temperature ranges from 20 to 200°C under a pressure of 50 mmHg in a non-contact-type distillation column where heat exchange is uniform and further distillation at temperatures increased by 20°C to discharge a low-molecular-weight volatile material (low-boiling-point material), subjected to secondary distillation at a temperature of 250°C for 10 min to discharge a middle- or low-boiling-point material, and cooled, followed by removing a carbonated fatty acid using a high-efficiency filter, thereby obtaining a fatty acid in which the amount of linolenic acid was increased to 82 wt% and the amount of low-molecular-weight high-volatile material was decreased to less than 2 wt%. This fatty acid was quantitatively added in an amount of 0.7 to 2.5 wt% to the PET DC of Comparative Example 1 in the same manner as in Comparative Example 1, thereby yielding POY and SDY.

During the production process, back-pressure hunting of the spinneret and single-yarn breakage at the outlet of the spinneret did not occur. The obtained filaments were improved in strength and elongation, compared to the filaments of Comparative Example 1, and especially, softness, dyeing property, and perspiration-absorbing and quick-drying properties were remarkably increased.

Table 6 below shows the physical properties of the obtained yarn.

The physical properties (DTY 75De) are given in Table 6 below.

**[Table 6]**

| | Strength (g/d) | Elongation (%) | Bulk | Dyeing (%) | Perspiration-absorbing and quick-drying | |
|---|---|---|---|---|---|---|
| | | | | | Drying speed (min) | Absorption speed (mm/10 min) |
| General yarn | 4.92 | 18 | 38 | 8 | 240 | 100 |
| Fatty acid-containing yarn (1.5%) | 4.70 | 23 | 39 | 16 | 190 | 200 |

### (Example 2)

Various types of woven and knitted fabrics were produced using the filaments of Example 1, and the obtained fabric had softness like that of rayon and an outer appearance similar to that of wool. Furthermore, the obtained fabric manifested excellent antistatic and dyeing properties.

### (Example 3)

The vegetable fatty acid used in Example 1 was quantitatively added in an amount of 1.5 wt% to the PETI polymer of Comparative Example 1 using a high-efficiency mixer, cooled and then solidified, thus obtaining chips.

The chips thus obtained were spun through a typical process to give filaments, and these filaments had the same properties as the filaments of Example 1.

### (Example 4)

The vegetable fatty acid used in Example 1 was quantitatively added in an amount of 1.5 wt% to the PETI polymer of Comparative Example 1 using a high-efficiency mixer, followed by continuous polymerization spinning, thus obtaining filaments. The filaments thus obtained had properties equal or superior to those of the filaments of Example 1.

### (Example 5)

This Example was performed in the same manner as Example 1, with the exception that chia seed oil having 80 wt% of linolenic acid and a low-molecular-weight volatile material of less than 2 wt% was used.

During the continuous production process, no problems occurred, and the properties of the obtained filaments were similar to those of the yarn of Example 1.

### (Comparative Example 2)

Onto the PET dry chip (PET DC) of Comparative Example 1, commercially available linseed oil having 56 wt% of linolenic acid and 5.1 wt% of a low-molecular-weight volatile material was uniformly and continuously sprayed in an amount of 0.7 wt% using a quantitative pump, thus producing a long polyester fiber as in Comparative Example 1.

30 min after the initiation of production, back-pressure hunting began to occur, and after 40 min, single-yarn breakage frequently occurred, and thus further production was not possible.

## Claims

1. A synthetic fiber or synthetic resin product, containing 0.7 to 2.5 wt% of a vegetable oil that comprises 70 wt% or more of linolenic acid and a low-molecular-weight volatile material having a boiling point of 120 to 220°C in an amount of less than 5 wt%.

2. The synthetic fiber or synthetic resin product of claim 1, wherein an amount of linolenic acid in the vegetable oil is 75 wt% or more.

3. The synthetic fiber or synthetic resin product of claim 1, wherein an amount of linolenic acid in the vegetable oil is 80 wt% or more.

4. The synthetic fiber or synthetic resin product of claim 1, wherein an amount of linolenic acid in the vegetable oil is 82 wt% or more.

5. The synthetic fiber or synthetic resin product of claim 1, wherein an amount of the low-molecular-weight volatile material having a boiling point of 120 to 220°C in the vegetable oil is less than 3 wt%.

6. The synthetic fiber or synthetic resin product of claim 1, wherein an amount of the low-molecular-weight volatile material having a boiling point of 120 to 220°C in the vegetable oil is less than 1 wt%.

7. The synthetic fiber or synthetic resin product of claim 1, wherein an amount of the low-molecular-weight volatile material having a boiling point of 120 to 220°C in the vegetable oil is less than 0.1 wt%.

8. A method of manufacturing a synthetic fiber containing linolenic acid, comprising:
polymerizing a fiber-forming polymer,
adding 0.7 to 2.5 wt% of a vegetable oil that comprises 70 wt% or more of linolenic acid and a low-molecular-weight volatile material having a boiling point of 120 to 220°C in an amount of less than 5 wt%, and
performing graft polymerization.

9. The method of claim 8, wherein an amount of linolenic acid in the vegetable oil is 75 wt% or more.

10. The method of claim 8, wherein an amount of linolenic acid in the vegetable oil is 80 wt% or more.

11. The method of claim 8, wherein an amount of linolenic acid in the vegetable oil is 82 wt% or more.

12. The method of claim 8, wherein an amount of the low-molecular-weight volatile material having a boiling point of 120 to 220°C in the vegetable oil is less than 3 wt%.

13. The method of claim 8, wherein an amount of the low-molecular-weight volatile material having a boiling point of 120 to 220°C in the vegetable oil is less than 1 wt%.

14. The method of claim 8, wherein an amount of the low-molecular-weight volatile material having a boiling point of 120 to 220°C in the vegetable oil is less than 0.1 wt%.

15. A method of manufacturing a synthetic resin containing linolenic acid, comprising:
polymerizing a fiber-forming polymer,
adding 0.7 to 2.5 wt% of a vegetable oil that comprises 70 wt% or more of linolenic acid and a low-molecular-weight volatile material having a boiling point of 120 to 220°C in an amount of less than 5 wt%, and
performing graft polymerization.

16. The method of claim 8, wherein an amount of linolenic acid in the vegetable oil is 75 wt% or more.

17. The method of claim 8, wherein an amount of linolenic acid in the vegetable oil is 80 wt% or more.

18. The method of claim 8, wherein an amount of linolenic acid in the vegetable oil is 82 wt% or more.

19. The method of claim 8, wherein an amount of the low-molecular-weight volatile material having a boiling point of 120 to 220°C in the vegetable oil is less than 3 wt%.

20. The method of claim 8, wherein an amount of the low-molecular-weight volatile material having a boiling point of 120 to 220°C in the vegetable oil is less than 1 wt%.

21. The method of claim 8, wherein an amount of the low-molecular-weight volatile material having a boiling point of 120 to 220°C in the vegetable oil is less than 0.1 wt%.

## Patentansprüche

1. Synthetische Faser oder synthetisches Harzprodukt, enthaltend 0,7 bis 2,5 Gew.-% von einem pflanzlichen Öl, das 70 Gew.-% oder mehr von Leinölsäure umfasst, und ein flüchtiges Material von niedrigem Molekulargewicht mit einem Siedepunkt von 120 bis 220°C in einer Menge von weniger als 5 Gew.-%.

2. Synthetische Faser oder synthetisches Harzprodukt nach Anspruch 1, wobei eine Menge von Leinölsäure in dem pflanzlichen Öl 75 Gew.-% oder mehr beträgt.

3. Synthetische Faser oder synthetisches Harzprodukt nach Anspruch 1, wobei eine Menge von Leinölsäure in dem pflanzlichen Öl 80 Gew.-% oder mehr beträgt.

4. Synthetische Faser oder synthetisches Harzprodukt nach Anspruch 1, wobei eine Menge von Leinölsäure in dem pflanzlichen Öl 82 Gew.-% oder mehr beträgt.

5. Synthetische Faser oder synthetisches Harzprodukt nach Anspruch 1, wobei eine Menge des flüchtigen Materials von niedrigem Molekulargewicht mit einem Siedepunkt von 120 bis 220°C in dem pflanzlichen Öl weniger als 3 Gew.-% beträgt.

6. Synthetische Faser oder synthetisches Harzprodukt nach Anspruch 1, wobei eine Menge des flüchtigen Materials von niedrigem Molekulargewicht mit einem Siedepunkt von 120 bis 220°C in dem pflanzlichen Öl weniger als 1 Gew.-% beträgt.

7. Synthetische Faser oder synthetisches Harzprodukt nach Anspruch 1, wobei eine Menge des flüchtigen Materials von niedrigem Molekulargewicht mit einem Siedepunkt von 120 bis 220°C in dem pflanzlichen Öl weniger als 0,1 Gew.-% beträgt.

8. Verfahren zur Herstellung einer synthetischen Faser enthaltend Leinölsäure, umfassend:
Polymerisation eines faserbildenden Polymers,
Zugabe von 0,7 bis 2,5 Gew.-% von einem pflanzlichen Öl, das 70 Gew.-% oder mehr von Leinölsäure umfasst, und einem flüchtigen Material von niedrigem Molekulargewicht mit einem Siedepunkt von 120 bis 220°C in einer Menge von weniger als 5 Gew.-%, und
Durchführen von Pfropfpolymerisation.

9. Verfahren nach Anspruch 8, wobei eine Menge von Leinölsäure in dem pflanzlichen Öl 75 Gew.-% oder mehr beträgt.

10. Verfahren nach Anspruch 8, wobei eine Menge von Leinölsäure in dem pflanzlichen Öl 80 Gew.-% oder mehr beträgt.

11. Verfahren nach Anspruch 8, wobei eine Menge von Leinölsäure in dem pflanzlichen Öl 82 Gew.-% oder mehr beträgt.

12. Verfahren nach Anspruch 8, wobei eine Menge des flüchtigen Materials von niedrigem Molekulargewicht mit einem Siedepunkt von 120 bis 220°C in dem pflanzlichen Öl weniger als 3 Gew.-% beträgt.

13. Verfahren nach Anspruch 8, wobei eine Menge des flüchtigen Materials von niedrigem Molekulargewicht mit einem Siedepunkt von 120 bis 220°C in dem pflanzlichen Öl weniger als 1 Gew.-% beträgt.

14. Verfahren nach Anspruch 8, wobei eine Menge des flüchtigen Materials von niedrigem Molekulargewicht mit einem Siedepunkt von 120 bis 220°C in dem pflanzlichen Öl weniger als 0,1 Gew.-% beträgt.

15. Verfahren zur Herstellung eines synthetischen Harzes enthaltend Leinölsäure, umfassend:
Polymerisation eines faserbildenden Polymers,
Zugabe von 0,7 bis 2,5 Gew.-% von einem pflanzlichen Öl, das 70 Gew.-% oder mehr von Leinölsäure umfasst, und einem flüchtigen Material von niedrigem Molekulargewicht mit einem Siedepunkt von 120 bis 220°C in einer Menge von weniger als 5 Gew.-%, und
Durchführen von Pfropfpolymerisation.

16. Verfahren nach Anspruch 8, wobei eine Menge von Leinölsäure in dem pflanzlichen Öl 75 Gew.-% oder mehr beträgt.

17. Verfahren nach Anspruch 8, wobei eine Menge von Leinölsäure in dem pflanzlichen Öl 80 Gew.-% oder mehr beträgt.

18. Verfahren nach Anspruch 8, wobei eine Menge von Leinölsäure in dem pflanzlichen Öl 82 Gew.-% oder mehr beträgt.

19. Verfahren nach Anspruch 8, wobei eine Menge des flüchtigen Materials von niedrigem Molekulargewicht mit einem Siedepunkt von 120 bis 220°C in dem pflanzlichen Öl weniger als 3 Gew.-% beträgt.

20. Verfahren nach Anspruch 8, wobei eine Menge des flüchtigen Materials von niedrigem Molekulargewicht mit einem Siedepunkt von 120 bis 220°C in dem pflanzlichen Öl weniger als 1 Gew.-% beträgt.

21. Verfahren nach Anspruch 8, wobei eine Menge des flüchtigen Materials von niedrigem Molekulargewicht mit einem Siedepunkt von 120 bis 220°C in dem pflanzlichen Öl weniger als 0,1 Gew.-% beträgt.

## Revendications

1. Produit de fibre synthétique ou de résine synthétique, contenant de 0,7 à 2,5% en poids d'une huile végétale qui comprend 70% en poids ou plus d'acide linolénique et une matière volatile de faible poids moléculaire ayant un point d'ébullition de 120 à 220°C en une quantité de moins de 5% en poids.

2. Produit de fibre synthétique ou de résine synthétique selon la revendication 1, dans lequel une quantité d'acide linolénique dans l'huile végétale est de 75% en poids ou plus.

3. Produit de fibre synthétique ou de résine synthétique selon la revendication 1, dans lequel une quantité d'acide linolénique dans l'huile végétale est de 80% en poids ou plus.

4. Produit de fibre synthétique ou de résine synthétique selon la revendication 1, dans lequel une quantité d'acide linolénique dans l'huile végétale est de 82% en poids ou plus.

5. Produit de fibre synthétique ou de résine synthétique selon la revendication 1, dans lequel une quantité de la matière volatile de faible poids moléculaire ayant un point d'ébullition de 120 à 220°C dans l'huile végétale est inférieure à 3% en poids.

6. Produit de fibre synthétique ou de résine synthétique selon la revendication 1, dans lequel une quantité de la matière volatile de faible poids moléculaire ayant un point d'ébullition de 120 à 220°C dans l'huile végétale est inférieure à 1% en poids.

7. Produit de fibre synthétique ou de résine synthétique selon la revendication 1, dans lequel une quantité de la matière volatile de faible poids moléculaire ayant un point d'ébullition de 120 à 220°C dans l'huile végétale est inférieure à 0,1% en poids.

8. Procédé de fabrication d'une fibre synthétique contenant de l'acide linolénique, comprenant :
la polymérisation d'un polymère formant des fibres,
l'ajout de 0,7 à 2,5% en poids d'une huile végétale qui comprend 70% en poids ou plus d'acide linolénique et un matériau volatil de faible poids moléculaire ayant un point d'ébullition de 120 à 220°C en une quantité inférieure à 5% en poids et
la mise en œuvre d'une polymérisation par greffage.

9. Procédé selon la revendication 8, dans lequel une quantité d'acide linolénique dans l'huile végétale est de 75% en poids ou plus.

10. Procédé selon la revendication 8, dans lequel une quantité d'acide linolénique dans l'huile végétale est de 80% en poids ou plus.

11. Procédé selon la revendication 8, dans lequel une quantité d'acide linolénique dans l'huile végétale est de 82% en poids ou plus.

12. Procédé selon la revendication 8, dans lequel une quantité de matière volatile de faible poids moléculaire ayant un point d'ébullition de 120 à 220°C dans l'huile végétale est inférieure à 3% en poids.

13. Procédé selon la revendication 8, dans lequel une quantité de matière volatile de faible poids moléculaire ayant un point d'ébullition de 120 à 220°C dans l'huile végétale est inférieure à 1% en poids.

14. Procédé selon la revendication 8, dans lequel une quantité de matière volatile de faible poids moléculaire ayant un point d'ébullition de 120 à 220°C dans l'huile végétale est inférieure à 0,1% en poids.

15. Procédé de fabrication d'une résine synthétique contenant de l'acide linolénique, comprenant :
la polymérisation d'un polymère formant des fibres,
l'ajout de 0,7 à 2,5% en poids d'une huile végétale qui comprend 70% en poids ou plus d'acide linolénique et un matériau volatil de faible poids moléculaire ayant un point d'ébullition de 120 à 220°C en une quantité inférieure à 5% en poids et
la mise en œuvre d'une polymérisation par greffage.

16. Procédé selon la revendication 8, dans lequel une quantité d'acide linolénique dans l'huile végétale est de 75% en poids ou plus.

17. Procédé selon la revendication 8, dans lequel une quantité d'acide linolénique dans l'huile végétale est de 80% en poids ou plus.

18. Procédé selon la revendication 8, dans lequel une quantité d'acide linolénique dans l'huile végétale est de 82% en poids ou plus.

19. Procédé selon la revendication 8, dans lequel une quantité de matière volatile de faible poids moléculaire ayant un point d'ébullition de 120 à 220°C dans l'huile végétale est inférieure à 3% en poids.

20. Procédé selon la revendication 8, dans lequel une quantité de matière volatile de faible poids moléculaire ayant un point d'ébullition de 120 à 220°C dans l'huile végétale est inférieure à 1% en poids.

21. Procédé selon la revendication 8, dans lequel une quantité de matière volatile de faible poids moléculaire ayant un point d'ébullition de 120 à 220°C dans l'huile végétale est inférieure à 0,1% en poids.
